# EUROPEAN PATENT APPLICATION

(11) **EP 3 649 935 A1**
(43) Date of publication of application: **13.05.2020**
(21) Application number: 19206922.7
(22) Date of filing: 04.11.2019
(51) Int. Cl.: A61B 5/083, A61B 5/00

(54) **BREATH TEST DEVICE**

(30) Priority: 08.11.2018 JP 2018210216
(71) Applicant: Japan Precision Instruments Inc., Shibukawa-shi Gumma 377-0293 (JP)
(72) Inventor: YOSHIDA, Akira, Shibukawa-shi, Gunma 377-0293 (JP); HIRASAKI, Yutaka, Shibukawa-shi, Gunma 377-0293 (JP); KONDO, Hideki, Shibukawa-shi, Gunma 377-0293 (JP)
(74) Representative: Betten & Resch

(57) **Abstract**

A breath test device provided with a main body part 20 to which a gas sensor for measuring an infrared absorptivity of a testee's breathing gas is communicably connected thereto, wherein the main body part 20 includes: a measuring part 21 which measures the concentration of carbon dioxide contained in the testee's exhaled breath based on the measurement result by the gas sensor for a period from the start to the end of a preset plurality of breathings which is taken as a measurement unit; a storage part 24 which stores the measurement result by the measuring part 21 at least for the measurement unit; a display part 25 which displays the measurement result by the measuring part 21 on a screen; and an screen control part 26 forming, as an image that the display part 25 is controlled to display, a display image including all of capnogram waveforms arranged side-by-side on the screen based on the contents stored in the storage part 24, each capnogram waveform representing time-dependent change in the carbon dioxide concentration for the measurement unit.

## Description

### BACKGROUND

### Technical field

The present invention relates to a breath test device which can measure the concentration of carbon dioxide contained in the exhaled breath.

### Description of Related Art

In recent years, monitoring EtCO₂ (End tidal CO₂) is sometimes conducted at a medical or nursing care site. EtCO₂, or end-tidal carbon dioxide concentration, is known to be highly correlated with arterial carbon dioxide partial pressure (PaCO₂) obtained from blood gas analysis. Therefore, monitoring EtCO₂ and estimating the change in PaCO₂ from the EtCO₂ enable non-invasive recognition of the ventilation state of the testee's respiratory system.

EtCO₂ is monitored using a breath test device called a capnometer. Examples of the breath test device include a device which utilizes a property of infrared ray of being absorbed by carbon dioxide and which is configured to measure the concentration of carbon dioxide contained in a testee's exhaled breath during a period for which the testee takes a preset plurality (for example 5 or more and 7 or less) of breaths and to display-output the measurement result (see, Patent Document 1).

### [Related Art Document]

### [Patent Document]

[Patent Document 1] Japanese Unexamined Patent Application Publication No. 2017-187365

### Brief Summary of the Invention

### Problems to be solved by the Invention

In the case of monitoring EtCO₂, a healthcare professional, a nursing care professional or the like recognizes a ventilation state of a testee's respiratory system referring to a display-output of a breath test device. Accordingly, it is desirable that the display-output of the breath test device can facilitate an easier and more appropriate recognition of the ventilation state of the testee's respiratory system.

However, a conventional breath test device does not always facilitate the easier and more appropriate recognition of the ventilation state of the testee's respiratory system. For example, a breath test device sometimes display-outputs a capnogram waveform representing a time-dependent change in the carbon dioxide concentration as a measurement result of the carbon dioxide concentration. In this regard, the capnogram waveform is hitherto provided as a sweep-display in general. Therefore, some timings or modes of the display-output may possibly make it difficult to appropriately analyze the capnogram waveform particularly when a period in which a testee breathes for a plurality of times is to be measured.

An object of the present invention is to provide a breath test device which can realize an easier and more appropriate recognition of a ventilation state of a testee's respiratory system.

### Means to solve the Problem

An aspect of the present invention is devised to achieve the above-described object and relates to a breath test device provided with a sensor probe having a gas sensor for measuring an infrared absorptivity of a testee's breathing gas, and a main body part to which the sensor probe is communicably connected,
wherein the main body part includes:
a measuring part which measures the concentration of carbon dioxide contained in the testee's exhaled breath based on the measurement result by the gas sensor, for a period from the start to the end of a preset plurality of breathings which is taken as a measurement unit;
a storage part which stores the measurement result by the measuring part for at least the measurement unit;
a display part which displays the measurement result by the measuring part on a screen; and
an screen control part which forms, as an image that the display part is controlled to display, a display image including all of the capnogram waveforms arranged side-by-side on the screen based on the contents stored in the storage part, each capnogram waveform representing time-dependent change in the carbon dioxide concentration for the measurement unit.

### Advantage of the Invention

According to the present invention, an easier and more appropriate recognition of a testee's ventilation state can be realized.

### Brief Description of the Drawings

FIG. 1 is a block diagram showing an exemplary schematic constitution of a breath test device according to an embodiment of the present invention.
FIG. 2 is an exploded perspective diagram showing an exemplary schematic constitution of a sensor probe in a breath test device according to an embodiment of the present invention.
FIG. 3 is a block diagram showing an exemplary functional constitution of a main body part of a breath test device according to an embodiment of the present invention.
FIG. 4 is an explanatory diagram schematically showing an exemplary appearance constitution of the main body part of a breath test device according to an embodiment of the present invention.
FIG. 5 is an explanatory diagram showing a use mode of a sensor probe in a breath test device according to an embodiment of the present invention.
FIG. 6 is an exemplary explanatory diagram showing a concept of a capnogram waveform obtained with a breath test device according to an embodiment of the present invention.
FIG. 7 is an explanatory diagram showing a specific example of a display image that the display part of the breath test device according to an embodiment of the present invention on a screen, in which: (a) is a diagram showing the state before the start of the measurement of the carbon dioxide concentration; (b) is a diagram showing the state during the measurement of the carbon dioxide concentration; and (c) is a diagram showing the display image at the time when the measurement of the carbon dioxide concentration is completed.
FIG. 8 is an explanatory diagram (No. 1) showing a specific example of changing a display mode of a display image by a screen control part of a breath test device according to an embodiment of the present invention, in which the time axis scale of the capnogram waveform in the display image is changed.
FIG. 9 is an explanatory diagram (No. 2) showing a specific example of display mode of changing a display image by a screen control part of a breath test device according to an embodiment of the present invention, in which a display reference position in the display image for arranging the capnogram waveforms side-by-side is changed.

### Detailed Description of the Invention

The breath test device according to the present invention will be hereinafter explained based on the drawings.

### <1. Exemplary constitution of breath test device>

The breath test device explained as an example in this embodiment is configured to measure the concentration of carbon dioxide contained in the testee's exhaled breath. More particularly, the device is configured to measure the concentration of carbon dioxide contained in the testee's exhaled breath while the testee takes the preset plurality of breaths utilizing a property of infrared ray of being absorbed by carbon dioxide and to display-output the measurement result.

An exemplary constitution of the breath test device will be hereinafter explained.

### (Entire constitution)

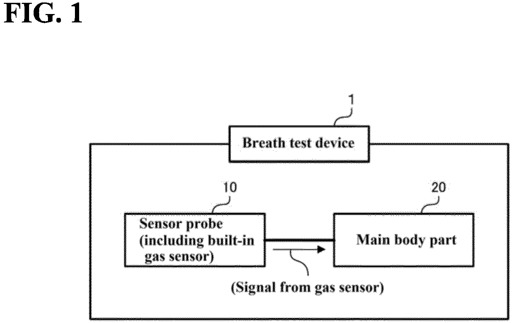

FIG. 1 is a block diagram showing an exemplary schematic constitution of a breath test device according to this embodiment.

The breath test device 1 shown in the figure is constituted generally to be provided with a sensor probe 10 and a main body part 20 which corresponds to a main body of the device. The sensor probe 10 is to be handled by the testee during the breath test. The main body part 20 is handled by an examiner (a healthcare professional, a nursing care professional or the like) during the breath test. The sensor probe 10 and the main body part 20 are communicably connected to each other. In this embodiment, the sensor probe 10 is connected to the main body part 20 via a cable, by way of an example.

### (Sensor probe)

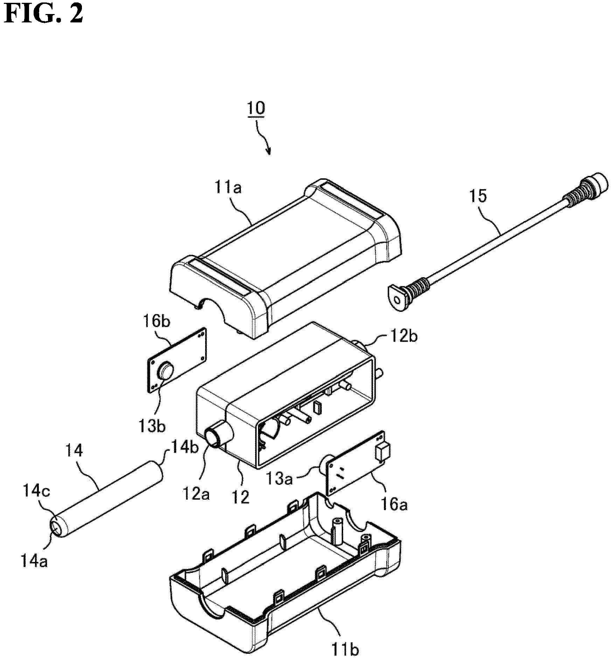

FIG. 2 is an exploded perspective diagram showing the exemplary schematic constitution of the sensor probe in the breath test device according to this embodiment.

The sensor probe 10 shown in the figure is constituted to include a case 11 (11a, 11b), an airway member 12 as a flow path forming member, gas sensors 13 (13a, 13b), a mouthpiece 14, and a cable 15.

The case 11 (11a, 11b) is constituted to house at least an airway member 12 and a gas sensor 13, and to be gripped by a testee while housing them. Although the case 11 (11a, 11b) may be considered to be formed of a material including, but not limited to, a resinous material such as an ABS (acrylonitrile-butadiene-styrene) resin, it may be formed of another material (e.g., a metallic material).

The airway member 12 forms a flow path for the testee's breathing gas (exhaled breath and inhaled breath). To this end, the airway member 12 is constituted to include a first tubular part 12a formed on the side of one end thereof and a second tubular part 12b formed on the side of the other end thereof, the first and second tubular parts being communicated with each other via a flow path, not shown, having an airtight structure. Note that, the flow path has a detection window formed thereon, not shown, which transmits the infrared ray, which will be described below. It is considered to form such an airway member 12, for example, by integral molding with a resinous material such as PET (polyethylene terephthalate) which has transparent hue.

The gas sensor 13 is constituted with a light emitting part 13a which radiates (emits) an infrared ray and a light receiving part 13b which receives the infrared ray. The gas sensor 13 may be mounted on the airway member 12 and may be integrated with the airway member 12. The term "integrated" used herein means that the airway member 12 and the gas sensor 13 are fixed to each other utilizing a physical coupling means (screw fastening, bonding etc.), forming a unified constitution as a whole.

A light emitting part 13a is mounted on a first sensor substrate 16a. In addition, the light receiving part 13b is mounted on a second sensor substrate 16b. The light emitting part 13a on the first sensor substrate 16a and the light receiving part 13b on the second sensor substrate 16b are placed to face each other across the flow path of the airway member 12. In addition, infrared ray radiated by the light emitting part 13a through the detection window toward the flow path is received by the light receiving part 13b through the detection window.

The mouthpiece 14 is constituted by a tubular shaped member that is to be fitted into a first tubular part 12a of the airway member 12, and is formed into a straw shape that opens at both longitudinal ends. The "straw shape" described here means the cylindrical shape with a central axis extending straight (linearly). The mouthpiece 14 has openings 14a, 14b respectively formed on the both end edges. The end edge on the front side (the side not to be fitted in the first tubular part 12a) of the mouthpiece 14 has a curved tapered part 14c rounded inward so that the opening 14a on the front side has a diameter smaller than the diameter of the opening 14b on the rear side (the side to be fitted in the first tubular part 12a) by the contraction in the opening diameter due to the curved tapered part 14c. Since the curved tapered part 14c is provided on one end of the mouthpiece 14, the roundness of the curved tapered part 14c makes it comfortable for the testee to hold the opening 14a side of the mouthpiece 14 in his/her mouth. In addition, when the testee breathes while holding the mouthpiece 14 in his/her mouth, his/her saliva is less likely to enter the inside of the mouthpiece 14. The mouthpiece 14 may be made of metal, resin, or paper.

The mouthpiece 14 is preferably disposable (for single use) and is detachably connected to the first tubular part 12a of the airway member 12. In this case, the mouthpiece 14 is preferably made of resin or paper from the viewpoint of cost and the like. In this embodiment, the mouthpiece 14 is constituted by an integral structure made of resin. In this case, a constituent material of the mouthpiece 14 is preferably polypropylene from the viewpoint of easy production, cost, and the like. In addition, the mouthpieces 14 are preferably provided in individual aseptic packages, each including several to a dozen of mouthpieces, and more preferably in individual aseptic packages, each including one mouthpiece. It is preferable to take out and use one mouthpiece from the packaging at a time.

The cable 15 electrically connects the sensor probe 10 (particularly, the first sensor substrate 16a and the second sensor substrate 16b in the sensor probe 10) to the main body part 20. The length of the cable 15 is set to an appropriate length as needed. The cable 15 is used for supplying power from the sensor probe 10 to the main body part 20 in order to drive the gas sensor 13, for delivering a control signal to control the drive of the gas sensor 13 between the main body part 20 and the sensor probe 10, or for outputting the results detected by the gas sensor 13 as an electrical signal.

### (Main body part)

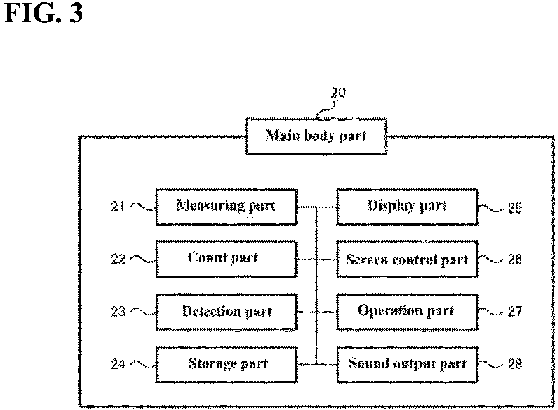

FIG. 3 is a block diagram showing an exemplary functional constitution of the main body part of the breath test device according to this embodiment.

The main body part 20 shown in the figure is constituted utilizing a computer which conducts predetermined information processing, and constituted to have functions as a measuring part 21, a count part 22, a detection part 23, a storage part 24, a display part 25, a screen control part 26, an operation part 27, and a sound output part 28.

The measuring part 21 measures the concentration of carbon dioxide contained in the testee's exhaled breath (EtCO₂) based on the measurement result by the gas sensor 13. In the measuring part 21, for example, an electrical signal output from the gas sensor 13 is subjected to signal processing based on a predetermined algorithm to convert the electrical signal into a numerical value (in mmHg) indicating the carbon dioxide concentration in the exhaled breath, thereby conducting measurement of the carbon dioxide concentration. The measurement of the carbon dioxide concentration is conducted for each of a predetermined cycle depending on the drive frequency of the gas sensor 13, for example.

Moreover, the measurement of the carbon dioxide concentration by the measuring part 21 is continuously conducted for a period from the start to the end of a preset plurality (i.e., N as described below) of breathings, which is taken as a measurement unit, as described in more detail below. In other words, for a period in which the testee takes N (N is an integer of 2 or more) breathings taken as a measurement unit, the measuring part 21 continuously measures the carbon dioxide concentration. The value of N is preferably 3 or more and 10 or less, more preferably 4 or more and 8 or less, and still more preferably 5 or more and 7 or less. In this embodiment, the case in which N is set to 6 will be explained as a particularly preferred example.

It should be noted that the carbon dioxide concentration value measured by the measuring part 21 is stored in the storage part 24 in association with the timing at which the value is obtained (time information etc.).

The count part 22 counts a breathing frequency of the testee. In the count part 22, the breathing frequency is counted using a waveform representing a time-dependent change in the level of the electrical signal output from the gas sensor 13 or a waveform representing a time-dependent change in carbon dioxide concentration (EtCO₂) measured by the measuring part 21 (hereinafter also referred to as a "capnogram waveform"). In this embodiment, the breathing frequency is counted using the capnogram waveform by way of an example. In that case, one breathing period (hereinafter also referred to as a "single breathing period") includes an exhalation period and an inhalation period, and the output waveform of the carbon dioxide concentration varies depending on the individual periods. Accordingly, the count part 22 can count the breathing frequency based on the change in the capnogram waveform.

The detection part 23 detects the maximum value of the carbon dioxide concentration when the testee breathes, based on the measurement result of the carbon dioxide concentration by the measuring part 21. In this detection part 23, the peak value which is the highest carbon dioxide concentration in the capnogram waveform appearing as a mountain-shape for each single breathing period is detected as the maximum value of the carbon dioxide concentration per single breathing. Since the value of the carbon dioxide concentration measured by the measuring part 21 for each breathing period is stored in the storage part 24, the detection part 23 detects the maximum value of the carbon dioxide concentration measured by the measuring part 21 at every count-up of the breathing frequency by the count part 22 during a single breathing period. Therefore, the maximum value of the carbon dioxide concentration per single breathing may be considered as the maximum value of the carbon dioxide concentration during a single breathing period.

Note that, when the frequency counted by the count part 22 reaches N (6 in this embodiment), the detection part 23 may compare the maximum values of the carbon dioxide concentration for each breathing to detect the maximum value of the carbon dioxide concentration during the period involving breathing frequency of N.

In addition, the detection part 23 may conduct an averaging procedure of the maximum values of the detected carbon dioxide concentration. In this embodiment, a moving average method is applied by way of an example. In this case, at every count-up of the breathing frequency counted by the count part 22, the detection part 23 averages the maximum values of the carbon dioxide concentration, detected up to the time, according to the moving average method.

The storage part 24 stores the measurement result by the measuring part 21. In the storage part 24, the carbon dioxide concentration value measured by the measuring part 21 is stored as the measurement result by the measuring part 21 in association with the timing at which the value is obtained (time information etc.).

It should be noted that, in the storage part 24, a memory capacity is saved which can store the measurement results by the measuring part 21 for at least one measurement unit (i.e., involving breathing frequency of N).

Moreover, the storage part 24 may store, in addition to the measurement result by the measuring part 21, the maximum value of the carbon dioxide concentration detected by the detection part 23, or an averaged value obtained by the averaging procedure by the detection part 23.

The display part 25 displays the measurement result by the measuring part 21 on a screen. The measurement result displayed on the screen by the display part 25 includes at least capnogram waveform which represents a time-dependent change in the carbon dioxide concentration (EtCO₂) as described in detail below. In addition, the measurement result displayed on the screen by the display part 25 may also include the maximum value or the averaged value of the carbon dioxide concentration detected by the detection part 23. The display part 25 may be constituted using a liquid crystal display, an organic EL display or the like.

The screen control part 26 controls a screen display by the display part 25. When the display part 25 displays the measurement result by the measuring part 21 on the screen, this screen control part 26 controls the display part 25 to form a display image required for the screen display, thereby controlling the screen display. To this end, the screen control part 26 is constituted to have a function to conduct an image processing for forming the display image. Specific examples of the display control by the screen control part 26 will be described in detail below.

The operation part 27 is operated by a user (i.e., a healthcare professional, a nursing care professional or the like who is an examiner of the breath test) who handles the main body part 20. To this end, the operation part 27 is constituted to have buttons for various operations.

The sound output part 28 outputs a predetermined sound (e.g., a buzzer sound) to the testee who handles the sensor probe 10 or the user who handles the main body part 20. Such a sound output part 28 may be constituted using a speaker, for example.

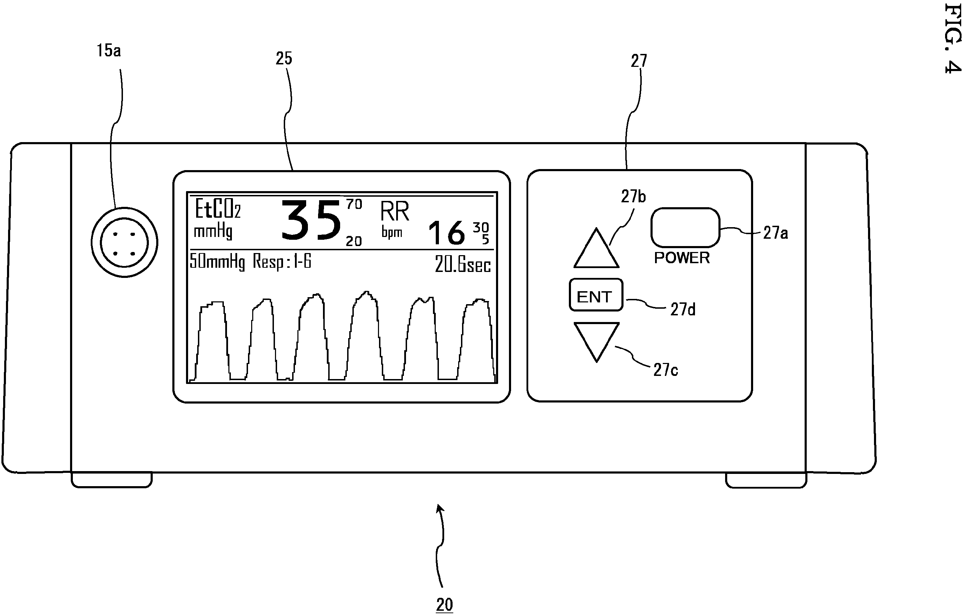

FIG. 4 is an explanatory diagram schematically showing an exemplary appearance constitution of the main body part of the breath test device according to this embodiment.

In the main body part 20 shown in the figure, the display part 25 having a display screen and an operation part 27 having an operation button, an operation switch or the like are arranged next to each other on the front face side of an enclosure facing the user who handles the main body part 20. In this regard, on the front face side of the enclosure of the main body part 20, a connector 15a for connecting the cable 15 of the sensor probe 10 may be arranged in addition to the display part 25 and the operation part 27.

In the operation part 27, at least a power button 27a to power-on/off the main body part 20, and an up button 27b, a down button 27c and an enter button 27d to be appropriately operated by the user of the main body part 20 are arranged. In the operation part 27, another button (not shown) may be further arranged.

The up button 27b, the down button 27c and the enter button 27d are used mainly for setting of the operation of the main body part 20 or for switching of the operation mode or the like. However, the up button 27b and the down button 27c are also used as operation buttons to change a display mode when the display part 25 conducts screen display, as described in detail below.

### <2. Exemplary processing operation of breath test device>

Next, an exemplary processing operation of the breath test device 1 having the above-described constitution will be explained.

### (Measurement of carbon dioxide concentration)

First, as a basic processing operation in a breath test device 1, a procedure of measuring the carbon dioxide concentration (EtCO₂) using the breath test device 1 will be explained.

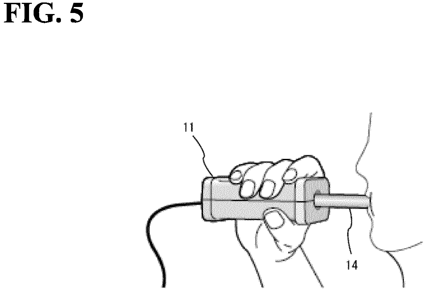

FIG. 5 is an explanatory diagram showing a use mode of the sensor probe in the breath test device according to this embodiment.

As shown in the figure, for the breath test using the breath test device 1, firstly, the testee who undergoes the breath test grasps the case 11 of the sensor probe 10 by his/her hand, and holds the opening 14a side of the mouthpiece 14 in his/her mouth. Then, while naturally breathing with the position being kept, the testee undergoes the breath test using the gas sensor 13.

When the testee breathes with the mouthpiece 14 held in his/her mouth, exhaled breath and inhaled breath flow alternately through the flow path in the airway member 12 of the sensor probe 10 according to the breathing of the testee. Under such circumstances, the gas sensor 13 emits infrared ray from the light emitting part 13a and receives the infrared ray at the light receiving part 13b, thereby detecting the carbon dioxide concentration in the exhaled breath flowing through the flow path. In this regard, carbon dioxide has a property of absorbing infrared ray radiated from the light emitting part 13a. Therefore, when the concentration of carbon dioxide which flows through the flow path is relatively higher, the proportion of infrared ray absorbed by carbon dioxide becomes greater accordingly. As a result, the amount of infrared ray received by the light receiving part 13b becomes relatively smaller. On the contrary, when the concentration of carbon dioxide which flows through the flow path is relatively lower, the proportion of infrared ray absorbed by carbon dioxide becomes smaller accordingly. As a result, the amount of infrared ray received by the light receiving part 13b becomes relatively greater. Therefore, the concentration of carbon dioxide contained in the testee's exhaled breath can be measured based on the amount of infrared ray received by the light receiving part 13b. For the measurement of the carbon dioxide concentration, for example, mid-infrared ray having a wavelength of 3.75 µm or more and 4.35 µm or less may be suitably used.

In the actual measurement, the electrical signal output from the light receiving part 13b is delivered to the main body part 20 via the cable 15. At this time, the processing described below will be conducted in the main body part 20.

In the main body part 20, the measuring part 21 firstly measures the concentration of carbon dioxide in the exhaled breath based on the electrical signal output from the gas sensor 13. Specifically, the measuring part 21 processes the electrical signal output from the gas sensor 13 according to a predetermined algorithm, so that the electrical signal is converted into a numerical value (in mmHg) indicating the carbon dioxide concentration in the exhaled breath (EtCO₂). Each time the carbon dioxide concentration value is obtained in such a way, the measuring part 21 stores the carbon dioxide concentration value in the storage part 24.

In addition, the count part 22 counts the testee's breathing frequency using a capnogram waveform representing the time-dependent change in the carbon dioxide concentration value.

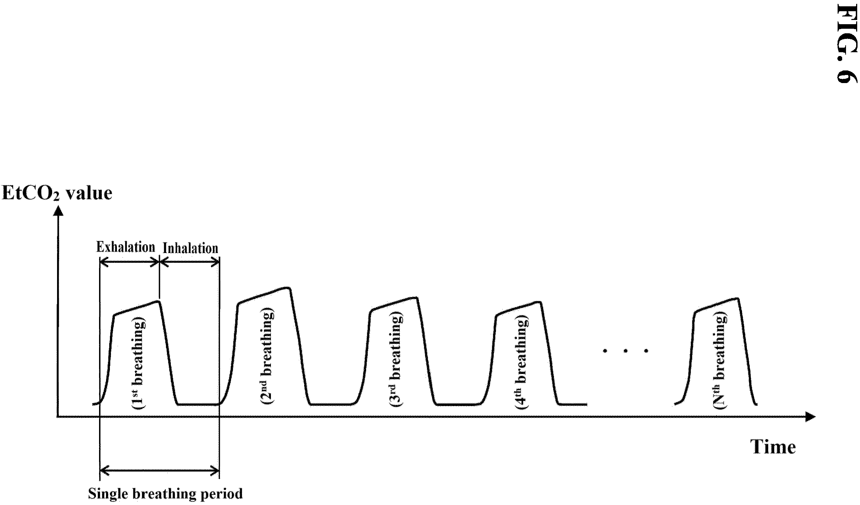

FIG. 6 is an explanatory diagram showing a concept of a capnogram waveform. In the illustrated example, the measured value of the carbon dioxide concentration (EtCO₂) is plotted in time series with the measured carbon dioxide concentration on the vertical axis and the time on the horizontal axis, thereby constituting a capnogram waveform.

When the testee actually repeats breathing, the capnogram waveform changes as follows. When the testee starts to exhale, the carbon dioxide concentration value increases rapidly and then tends to gradually increase. Next, when the testee starts to inhale, the carbon dioxide concentration value decreases rapidly. For this reason, the capnogram waveform exhibits a mountain-shaped waveform, and such a mountain-shaped waveform appears continuously in accordance with the testee's breathing frequency.

In addition, under the circumstances before the testee starts breathing, the measured value of the carbon dioxide concentration based on the electrical signal from the gas sensor 13 is 0 mmHg or near offer (several mmHg). Therefore, the count part 22 detects as a timing of exhalation start, for example, a timing when the carbon dioxide concentration value measured by the measuring part 21 exceeds a predetermined value (e.g., 20 mmHg), and then detects as a timing of inhalation end, for example, a timing when the carbon dioxide concentration value falls to or below the predetermined value. Then, the period from the timing of exhalation start to the timing of inhalation end is regarded as a "single breathing period", and such a single breathing period (in other words, a single mountain-shaped waveform) is counted as a single breathing. As a result, every time the testee breathes, the breathing frequency counted by the count part 22 is increased one by one.

In this case, each time an additional breathing frequency is counted by the count part 22, the detection part 23 detects the maximum value of the carbon dioxide concentration for each breathing period. For example, when the breathing frequency counted by the count part 22 is 1, the maximum value of the carbon dioxide concentration during a single breathing period is detected based on the measurement result of the carbon dioxide concentration obtained by the measuring part 21 for the first breathing. The same applies to the second and the subsequent breathings. As a result, the maximum value of the carbon dioxide concentration per breathing for each breathing frequency counted by the count part 22 is detected by the detection part 23.

Moreover, the detection part 23 may detect the maximum value of the carbon dioxide concentration during a period involving breathing frequency of N by comparing the maximum value of the carbon dioxide concentration in each breathing. Furthermore, the detection part 23 may conduct an averaging procedure of the maximum values of the detected carbon dioxide concentration.

Each time such a detection result is obtained, the detection part 23 controls the storage part 24 to store the detection result therein.

Then, when the breathing frequency counted by the count part 22 reaches the preset N (6 in this embodiment), the measuring part 21 and the detection part 23 determine that the processing for a single measurement unit is completed.

When processing for the single measurement unit is completed, the display part 25 displays the display image formed by the screen control part 26 on the screen as the measurement result for the measurement unit. In other words, at the time when the measurement for the measurement unit by the measuring part 21 is completed, the screen control part 26 forms a display image representing the measurement result for the measurement unit and controls the display part 25 to display the display image. The display image that the display part 25 displays on the screen will be described in detail later.

At this time when the processing for the single measurement unit is completed, the sound output part 28 may conduct sound output informing of the completion of the measurement utilizing a predetermined sound (e.g. a buzzer sound). With such a sound output being conducted, the testee, the examiner and the like involved in the breath test can be informed of the end of the test. Therefore, it is very convenient for the testee, the examiner and the like involved in the breath test since he/she can be easily and appropriately informed of the end of the test even without visually observing the screen display on the display part 25.

As described above, the measurement unit is defined as a period from the start to the end of the preset N (6 in this embodiment) breathings when the concentration of carbon dioxide contained in the testee's exhaled breath (EtCO₂) is to be measured in the main body part 20. This enables measurement of the carbon dioxide concentration that exhibits higher correlation with the carbon dioxide concentration in arterial blood. The reason is as follows.

For example, if the testee is a person who does not have a disease in the respiratory system, his/her way of breathing tends to be appropriately deep and stable, so that variation observed in the capnogram waveform becomes smaller. On the other hand, if the testee is a patient suffering from chronic obstructive pulmonary disease (hereinafter also referred to as "COPD"), his/her way of breathing tends to be shallow and unstable, so that variation is observed in the capnogram waveform at a higher probability. Therefore, if the maximum value of the carbon dioxide concentration per breathing can be recognized for N breathings, the measurement result of the carbon dioxide concentration can be obtained which exhibits a higher correlation with the carbon dioxide concentration in arterial blood. In other words, using a period from the start to the end of N breathings taken as a measurement unit, COPD can be more accurately diagnosed even without measuring the carbon dioxide concentration in arterial blood.

In particular, it has been found that, for the testee who is a patient suffering from COPD, about 6 times of breathings may include at least one relatively deeper breathing, in which the maximum concentration value of the carbon dioxide contained in the exhaled breath has strong correlation with the carbon dioxide concentration in the arterial blood. Therefore, it is preferable that the value of N is set to around 6 (5 or more and 7 or less). With this arrangement, the measurement result of the carbon dioxide concentration which has a stronger correlation with the carbon dioxide concentration in the arterial blood can be obtained in a shorter test period (breathing frequency).

### (Display image indicating measurement result)

Next, the display image displayed on a screen by the display part 25 as the measurement result of the carbon dioxide concentration will be explained with a specific example.

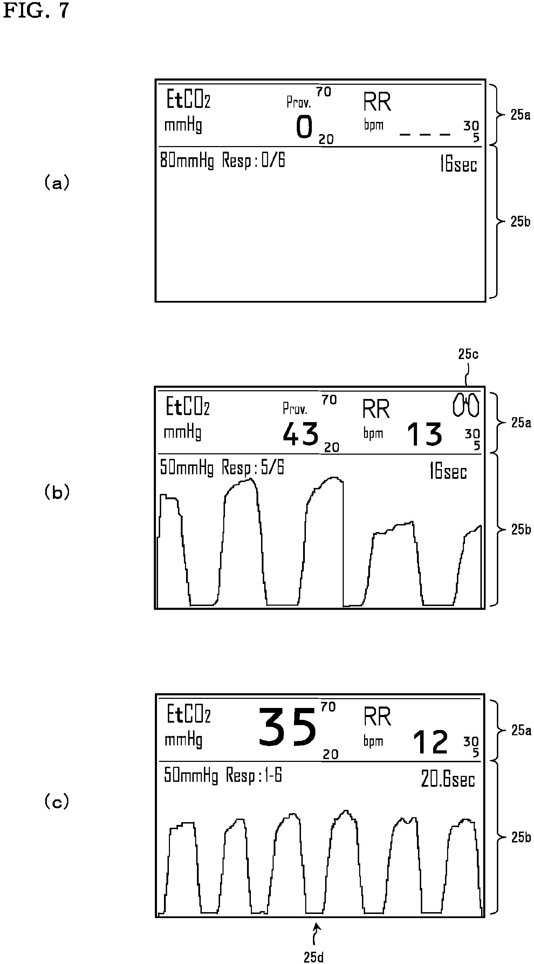

FIG. 7 is an explanatory diagram showing a specific example of the display image.

Before the start of the measurement of the carbon dioxide concentration by the breath test, the display screen of the display part 25 displays the display state shown in FIG. 7(a), for example. In other words, on the display screen shown in the figure, a display area 25a and a display area 25b are allocated for the carbon dioxide concentration value and for the capnogram waveform, respectively, but nothing is displayed for particular measurement results or the like since the measurement of the carbon dioxide concentration is not started yet.

Thereafter, during measurement of the carbon dioxide concentration after measurement of the breath test is started, the display screen of the display part 25 transitions to the display state shown in FIG. 7(b), for example. Namely, on the display screen shown in the figure, the value of the carbon dioxide concentration (EtCO₂) measured by the measuring part 21 is displayed in the carbon dioxide concentration display region 25a, while a capnogram waveform which is obtained by plotting the carbon dioxide concentration value in time series is displayed in the capnogram waveform display area 25b. However, the display on the display screen (in particular, the display of the capnogram waveform) at this time is a sweep-display in which the displayed content varies with time since the measurement of the carbon dioxide is in progress. In this regard, the time axis scale of the sweep-displayed capnogram waveform is considered to be defined in advance by default according to the driving frequency of the gas sensor 13, for example.

In addition, during measurement of the carbon dioxide concentration, N (six in this embodiment) breathings are taken so that the exhalation period and the inhalation period are alternately repeated. For this reason, an icon image 25c which can make the exhaled breath measurement state and the inhaled breath measurement state in the sensor probe 10 distinguishable from each other may be displayed on the display screen of the display part 25 during measurement of the carbon dioxide concentration. Specifically, the icon image 25c is lit on in the exhaled breath measurement state, and the icon image 25c is lit off in the inhaled breath measurement state so that these states can be distinguished from each other. Regarding the icon image 25c, after the measuring part 21 measures the carbon dioxide concentration value based on the measurement result by the gas sensor 13, it recognizes the timing of exhalation start and the timing of inhalation end according to the value, thereby the icon image 25c can be formed and displayed. With such an icon image 25c being displayed, the exhaled breath measurement state can be distinguished from the inhaled breath measurement state and the progress of the breath test can be easily and appropriately informed during the breath test, which makes it very convenient for the testee, the examiner and the like involved in the breath test.

By the way, the content displayed on the display screen of the display part 25 is presented to the user of the main body part 20 (i.e., a healthcare professional, a nursing care professional or the like who is an examiner of the breath test) so that he/she can recognize the ventilation state of the testee's respiratory system. Accordingly, it is desired to have a content displayed on the display screen that facilitates an easier and more appropriate recognition of the ventilation state of the testee's respiratory system. Specifically, in order to recognize the ventilation state of the testee's respiratory system, for example, the capnogram waveform is analyzed to determine the suitability of the state of an alveolar phase (plateau), the waveform variation for each breathing, or the like in some cases. The analysis of the capnogram waveform is required to be easily and appropriately conducted.

However, in the case where a period from the start to the end of N (6 in this embodiment) breathings is particularly taken as a measurement unit, some timings or modes of the display-output may possibly make it difficult to appropriately analyze the capnogram waveform when the capnogram waveform is sweep-displayed.

Therefore, in the main body part 20 of this embodiment, when a measurement of a single measurement unit involving N breathings is completed since the start of the measurement of the carbon dioxide concentration by the breath test, a display image 25d shown in FIG. 7(c) is display-output on the display screen of the display part 25, for example. The display image 25d shown in the figure includes the maximum value of the carbon dioxide concentration displayed in the display area 25a and, as a capnogram waveform displayed in the display area 25b, capnogram waveforms (mountain-shape waveforms) which respectively correspond to each of N breathing periods constituting a single measurement unit, all of the capnogram waveforms being sequentially arranged side-by-side in time series on the screen. Specifically, in the case where six breathings are taken as a single measurement unit, the timing of exhalation start in the first breathing is taken as a display reference position (i.e., near the leftmost position that is to be a reference when the display area 25b is displayed), and the respective capnogram waveforms are arranged so that all capnogram waveforms including that obtained in the first breathing to that obtained in the sixth breathing are sequentially arranged side-by-side from the display reference position in time series, thereby constituting a capnogram waveform for a single measurement unit. In the display image 25d, since all of the capnogram waveforms for the single measurement unit are arranged side-by-side, the state of the alveolar phase (plateau) in the waveform, waveform variation for each breathing and the like become apparent at a glance, and, as a result, the capnogram waveform can be easily and appropriately analyzed.

The display image 25d is formed by the screen control part 26 based on the content stored in the storage part 24. Namely, the screen control part 26 forms the above-described display image 25d when the measurement by the measuring part 21 is completed for the single measurement unit, and controls the display part 25 to display the formed display image 25d. Since the storage part 24 stores the measurement result by the measuring part 21 for at least the single measurement unit, the display image 25d may also be displayed even after the measurement for the measurement unit is completed.

However, when the display image 25d is to be formed, it is considered that simply arranging the capnogram waveforms side-by-side may require further improvement in order to facilitate an easier and more appropriate analysis of the capnogram waveform depending on the relation between the waveform and the size of the display screen of the display part 25 which displays the display image 25d. Therefore, in the main body part 20 according to this embodiment, the screen control part 26 conducts the following process when the display image 25d is to be formed.

The time required for a testee to take N breathings varies depending on the testee. In other words, the time required to measure the carbon dioxide concentration (EtCO₂) for a single measurement unit varies for each testee. Accordingly, when the capnogram waveforms are simply arranged side-by-side while the time axis scale stays at its default setting, all of the capnogram waveforms may not be displayed in the display screen or, on the contrary, there may be empty space on the display screen.

As such, in this embodiment, the screen control part 26 adjusts the time axis scale of the capnogram waveform in the display image 25d to be formed depending on the time required for N breathings which constitute a single measurement unit when the display image 25d that the display part 25 is controlled to display is to be formed. Specifically, since the time required for a single measurement unit is known at the time when the measurement for the single measurement unit is completed, the screen control part 26 forms the display image 25d while adjusting the time axis scale so that the required time becomes a full-scale value.

Since the screen control part 26 has such a function, the capnogram waveform for a single measurement unit is displayed in full scale in the time axis direction on the display screen of the display part 25 which is very preferable for facilitating an easier and more appropriate analysis of the capnogram waveform.

Furthermore, the magnitude of the carbon dioxide concentration value obtained by measuring the carbon dioxide concentration (EtCO₂) also varies from testee to testee. Therefore, it may become difficult to clearly display the state of the alveolar phase (plateau) in the waveform (particularly the state of waveform change) when the capnogram waveform is displayed while a concentration range stays at its default setting.

In this embodiment, when the display image 25d that the display part 25 is controlled to display is to be formed, the screen control part 26 adjusts the concentration range of the capnogram waveform in the display image 25d to be formed depending on the measurement result of the carbon dioxide concentration by the measuring part 21 (i.e., the magnitude of the measured carbon dioxide concentration value). Specifically, since the maximum value of the carbon dioxide concentration is known at the time when the measurement for a single measurement unit is completed, the screen control part 26 selects a reference range value corresponding to the maximum value from the preset plurality of reference range values and forms the display image 25d while adjusting the concentration range so that the selected reference range value is to be a full-scale value.

Since the screen control part 26 has such a function, the capnogram waveform for the single measurement unit is displayed in full-scale in the concentration value direction on the display screen of the display part 25. Accordingly, it is very suitable for facilitating easier and more appropriate analysis of the capnogram waveform to be facilitated.

### (Change of display mode)

As described above, in the main body part 20 of this embodiment, a display image 25d is formed in which all the capnogram waveforms for a single measurement unit are arranged side-by-side and the display image 25d is displayed on the display screen of the display part 25 to facilitate an easier and more appropriate analysis of the capnogram waveform.

However, when the capnogram waveform is to be analyzed, it is necessary to focus on a part of the capnogram waveform in some cases.

Therefore, in the main body part 20 of this embodiment, the screen control part 26 is able to change the display mode of the display image 25d that the display part 25 is controlled to display, in response to the operation of the operation part 27 by the user of the main body part 20 as described below.

### (Change of time axis scale)

Here, the case in which the time axis scale of the capnogram waveform in the display image 25d is changed will be firstly explained as a specific example of the change of the display mode of the display image 25d.

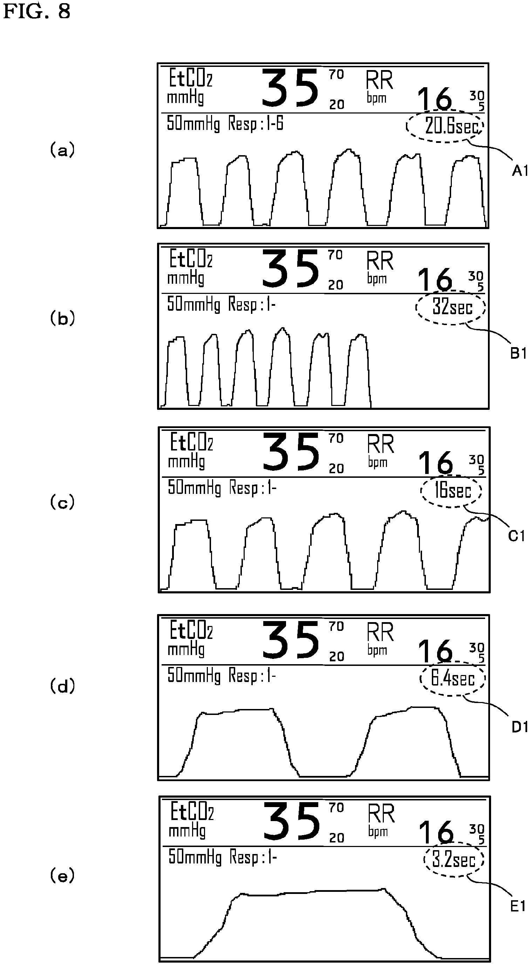

FIG. 8 is a diagram showing a specific example in which the time axis scale of the capnogram waveform in the display image is changed in this embodiment.

FIG. 8(a) shows an example of a display image 25d displayed by the display part 25 when the measurement by the measuring part 21 is completed for the single measurement unit. In the display image 25d shown in the figure, the time required for a testee to take N (six in this embodiment) breathings is "20.6 sec", and hence the time axis scale is adjusted so that the time required therefor becomes a full-scale (See A1 in the figure).

When the user of the main body part 20 presses the up button 27b of the operation part 27 while the display image 25d is displayed, the display mode of the display image 25d on the display part 25 transitions to a mode shown in FIG. 8(b). In the display mode shown in the figure, the time axis scale of the capnogram waveform in the display image 25d is one of a plurality of previously prepared time axis scales, in which "32 sec" is taken as a full-scale (see B1 in the figure).

In this state, when the user of the main body part 20 again presses the up button 27b of the operation part 27, the display mode of the display image 25d in the display part 25 transitions to the mode shown in FIG. 8(c). In the display mode shown in the figure, the time axis scale of the capnogram waveform in the display image 25d is one of a plurality of previously prepared time axis scales, in which "16 sec" is taken as a full-scale (see C1 in the figure).

In this state, when the user of the main body part 20 again presses the up button 27b of the operation part 27, the display mode of the display image 25d in the display part 25 transitions to the mode shown in FIG. 8(d). In the display mode shown in the figure, the time axis scale of the capnogram waveform in the display image 25d is one of a plurality of previously prepared time axis scales, in which "6.4 sec" is taken as a full-scale (see D1 in the figure).

In this state, when the user of the main body part 20 again presses the up button 27b of the operation part 27, the display mode of the display image 25d in the display part 25 transitions to the mode shown in FIG. 8(e). In the display mode shown in the figure, the time axis scale of the capnogram waveform in the display image 25d is one of a plurality of previously prepared time axis scales, in which "3.2 sec" is taken as a full-scale (see E1 in the figure).

In this state, when the user of the main body part 20 again presses the up button 27b of the operation part 27, the display mode of the display image 25d in the display part 25 returns to the mode shown in FIG. 8(a).

As described above, the display mode of the display image 25d on the display part 25 transitions for each operation of the up button 27b as follows: FIG. 8(a) → FIG. 8(b) → FIG. 8(c) → FIG. 8(d) → FIG. 8(e) → FIG. 8(a).

In other words, the screen control part 26 that controls the screen display by the display part 25 changes the time axis scale of the capnogram waveform in the display image 25d each time the up button 27b is pressed. When the time axis scale is to be changed, any one of a plurality of previously prepared time axis scales is selectively applied in response to the operation of the operation part (specifically, each time the up button 27b is pressed). At that time, the screen control part 26 changes the time axis scale of the capnogram waveform without shifting the display reference position of the capnogram waveform (in the figure, the position at the timing of exhalation start of the first breathing).

The time axis scale can be changed in such a mode that the transition is cyclically repeated in response to the press of the up button 27b since the storage part 24 stores the measurement result by the measuring part 21 for at least one measurement unit.

Although particular cases are explained herein as an example, in which a plurality of previously prepared time axis scales are "32 sec", "16 sec", "6.4 sec" or "3.2 sec", in addition to the time axis scale adjusted according to the time required for a single measurement unit, the plurality of time axis scales are not necessarily limited thereto and may be acceptable so long as they are appropriately set, for example, according to the driving frequency of the gas sensor 13, the processing capacity of the screen control part, or the like.

Further, when the time axis scale is to be changed, the screen control part 26 may conduct the image processing as follows. For example, in a case in which the default setting of the time axis scale is "16 sec", the screen control part 26 may conduct an image processing of decimating pixel data constituting a default capnogram waveform as necessary in transition to a mode in which "32 sec" is a full scale. In addition, for example, the screen control part 26, in transition to a mode in which "6.4 sec" or "3.2 sec" is a full scale, may conduct the image processing based on the pixel data constituting the default capnogram waveform while interpolating the insufficient pixel data as necessary. The screen control part 26 having a function of conducting such image processing can enhance picture quality of the image to be displayed after transition of the display mode while preventing the storage part 24 from requiring excessive storage capacity even when the time axis scale is changed.

As described above, in the main body part 20 of this embodiment, the time axis scale of the capnogram waveform in the display image 25d is changed in response to the press of the up button 27b. Accordingly, for example, displaying all of the capnogram waveforms for a single measurement unit side-by-side or displaying the enlarged mountain-shaped waveform constituting the capnogram waveform can be selectively conducted in response to the operation of the up button 27b. Therefore, it is convenient for the user of the main body unit 20 that analyzes the capnogram waveform, and is very suitable for easy and appropriate analysis of the capnogram waveform.

### (Changing display reference position)

Subsequently, as another specific example of changing the display mode of the display image 25d, a case in which the display reference position is changed when the capnogram waveform is arranged side-by-side in the display image 25d will be exemplified and explained.

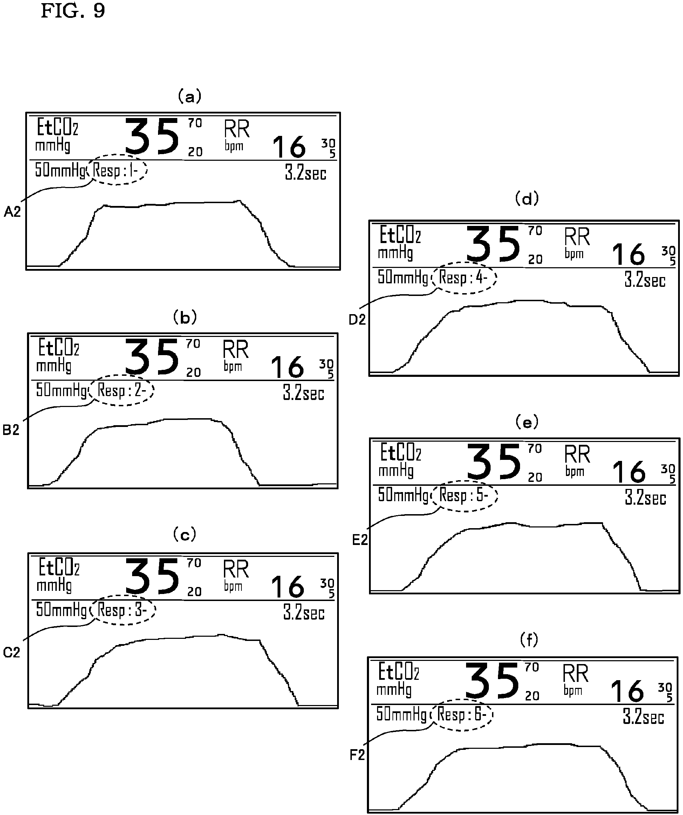

FIG. 9 is a diagram showing a specific example in which the display reference position is changed when the capnogram waveform is arranged side-by-side in the display image in this embodiment

The example in the figure represents a case in which a time axis scale with "3.2 sec" being a full scale is applied.

FIG. 9(a) illustrates an example in which a capnogram waveform (a mountain-shaped waveform obtained in the first breathing) is displayed with the timing of exhalation start of the first breathing as the display reference position (See A2 in the figure).

In the state with such a display, when the user of the main body part 20 presses the down button 27c of the operation part 27, the display mode in the display part 25 transitions to the mode shown in FIG. 9(b). The display mode shown in the figure displays a capnogram waveform (a mountain-shaped waveform obtained in the second breathing) with the timing of exhalation start of the second breathing as the display reference position (See B2 in the figure). In other words, the entire capnogram waveform is shifted so as to be displayed while the waveform rising position, which is the timing of exhalation start of the second breathing, is taken as the display reference position.

In this state, when the user of the main body part 20 again presses the down button 27c of the operation part 27, the display mode of the display image 25d in the display part 25 transitions to the mode shown in FIG. 9(c). The display mode shown in the figure displays a capnogram waveform (a mountain-shaped waveform obtained in the third breathing) with the timing of exhalation start of the third breathing as the display reference position (See C2 in the figure). In other words, the entire capnogram waveform is shifted so as to be displayed while the waveform rising position, which is the timing of exhalation start of the third breathing, is taken as the display reference position.

In this state, when the user of the main body part 20 again presses the down button 27c of the operation part 27, the display mode of the display image 25d in the display part 25 transitions to the mode shown in FIG. 9(d). The display mode shown in the figure displays a capnogram waveform (a mountain-shaped waveform obtained in the fourth breathing) with the timing of exhalation start of the fourth breathing as the display reference position (See D2 in the figure). In other words, the entire capnogram waveform is shifted so as to be displayed while the waveform rising position, which is the timing of exhalation start of the fourth breathing, is taken as the display reference position.

In this state, when the user of the main body part 20 again presses the down button 27c of the operation part 27, the display mode of the display image 25d in the display part 25 transitions to the mode shown in FIG. 9(e). The display mode shown in the figure displays a capnogram waveform (a mountain-shaped waveform obtained in the fifth breathing) with the timing of exhalation start of the fifth breathing as the display reference position (See E2 in the figure). In other words, the entire capnogram waveform is shifted so as to be displayed while the waveform rising position, which is the timing of exhalation start of the fifth breathing, is taken as the display reference position.

In this state, when the user of the main body part 20 again presses the down button 27c of the operation part 27, the display mode of the display image 25d in the display part 25 transitions to the mode shown in FIG. 9(f). The display mode shown in the figure displays a capnogram waveform (a mountain-shaped waveform obtained in the sixth breathing) with the timing of exhalation start of the sixth breathing as the display reference position (See F2 in the figure). In other words, the entire capnogram waveform is shifted so as to be displayed while the waveform rising position, which is the timing of exhalation start of the sixth breathing, is taken as the display reference position.

In this state, when the user of the main body part 20 again presses the down button 27c of the operation part 27, the display mode of the display image 25d in the display part 25 returns to the mode shown in FIG. 9(a).

Thus, the display mode of the display image 25d on the display part 25 transitions for each operation of the down button 27c as follows: FIG. 9(a) → FIG. 9(b) → FIG. 9(c) → FIG. 9(d) → FIG. 9(e) → FIG. 9(f) → FIG. 9(a).

In other words, the screen control part 26 that controls the screen display by the display part 25 changes the display reference position when the capnogram waveforms are arranged side-by-side in the display image 25d each time the down button 27c is pressed. Specifically, the screen control part 26 selectively applies, as the display reference position, any one of the waveform rising positions of each capnogram waveform corresponding to each of N breathings constituting the measurement unit (i.e., the position at the timing of exhalation start) in response to the operation of the down button 27c. Then, according to the selectively applied display reference position, the order of the mountain-shaped waveforms of the capnogram waveform respectively corresponding to each of N breathings is rearranged. Thereby, the mountain-shaped waveform applied as the display reference position is to be placed at the top position of the arrangement sequence.

In view of the foregoing, in the main body part 20 of this embodiment, the time axis scale of the capnogram waveform in the display image 25d is changed in response to the press of the down button 27c, in addition to the time axis scale of the capnogram waveform being changeable as described above. Therefore, for example, when the display reference position for arranging the capnogram waveforms side-by-side is changed while the time axis scale for enlarged display of the waveform being selected, the display part 25 can be controlled to conduct enlarged display of any one of the capnogram waveforms respectively corresponding to each of the N breathings (mountain-shaped waveform) constituting a single measurement unit, in response to the operation of the down button 27c. Therefore, it is very convenient for the user of the main body part 20 who analyzes the capnogram waveform particularly in the case of determining the state of the alveolar phase (plateau) of the waveform, and is very suitable for easy and appropriate analysis of the capnogram waveform.

Although a particular case is herein exemplified in which the display reference position for arranging the capnogram waveform side-by-side is changed while the time axis scale with "3.2 sec" being taken as a full scale is applied, the time axis scale and the display reference position can be changed separately and independently. For example, even when a time axis scale adjusted according to the time required for a single measurement unit is applied (for example, the state shown in FIG. 7(c) or FIG. 8(a)), the display reference position for arranging the capnogram waveforms side-by-side can be changed if the down button 27c is operated. In this case, the screen control part 26 controls the display part 25 to display the capnogram waveform in which the order of the mountain-shaped waveforms of the capnogram waveform is rearranged while changing the display reference position in response to the operation of the down button 27c.

### <3. Effect of this embodiment>

According to this embodiment, one or more effects described below may be obtained.
(a) In this embodiment, the concentration of carbon dioxide (EtCO₂) contained in the exhaled breath of the testee is measured using a period from the start to the end of N breathings as a single measurement unit, while a display image 25d is display-output as the measurement result, in which all of the capnogram waveforms respectively corresponding to each of N breathings constituting the measurement unit are arranged side-by-side on the screen. In the display image 25d, since all of the capnogram waveforms for the single measurement unit are arranged side-by-side, the state of the alveolar phase (plateau) in the waveform, waveform variation for each breathing and the like become apparent at a glance, and, as a result, the capnogram waveform can be easily and appropriately analyzed. In other words, according to this embodiment, even when the period from the start to the end of N breathings is taken as a single measurement unit, the capnogram waveform can be appropriately analyzed for the measurement unit. Accordingly, when the ventilation state of the testee's respiratory system is to be recognized, the easier and more appropriate recognition can be facilitated.
   In this embodiment, since a period from the start to the end of N (the value of N is preferably 6 or around 6, that is, 5 or more and 7 or less) times of breathings is taken as a measurement unit, COPD can be more accurately diagnosed even without measuring the concentration of carbon dioxide in arterial blood.
   In addition, even when the testee takes N breathings, since the storage part 24 stores the measurement result at least for the single measurement unit, a display image 25d, in which all the capnogram waveforms are arranged side-by-side, can be formed after the measurement for the single measurement unit is completed,. That is, storing the measurement results at least for the single measurement unit can consequently enable realization of an easier and more appropriate analysis of the capnogram waveform.
(b) In this embodiment, at the time when the measurement by the measuring part 21 is completed for the single measurement unit, the display part 25 displays a display image 25d in which all the capnogram waveforms for the measurement unit are arranged side-by-side. For this reason, the display image 25d is displayed without requiring an operation by the user of the main body part 20, which offers excellent convenience to the user of the main body part 20.
(c) In this embodiment, when the display image 25d is to be displayed, the time axis scale of the capnogram waveform in the display image is adjusted according to the time required for N breathings constituting a single measurement unit. In other words, even if the time required for N breathings varies depending on the testee, a capnogram waveform for the single measurement unit is displayed in full scale in the time axis direction on the display screen of the display part 25. Therefore, it is very suitable for facilitating an easier and more appropriate analysis of the capnogram waveform.
(d) In this embodiment, when the display image 25d is to be displayed, the concentration range of the capnogram waveform in the display image 25d is adjusted according to the measurement result of the carbon dioxide concentration (EtCO₂) by the measuring part 21. In other words, even when the magnitude of the carbon dioxide concentration value obtained by measurement varies depending on the testee, the capnogram waveform for the single measurement unit is displayed in full scale in the direction of the concentration value on the display screen of display part 25. Therefore, it is very suitable for facilitating an easier and more appropriate analysis of the capnogram waveform.
(e) In this embodiment, the display mode of the display image 25d which the display part 25 is controlled to display can be changed in response to the operation of the operation part 27 by the user of the main body part 20. Therefore, for example, when the capnogram waveform is to be analyzed, it is possible to easily and flexibly cope with, for example, focusing on a part of the capnogram waveform. As the result, it is very suitable for facilitating an easier and more appropriate analysis of the capnogram waveform.
(f) In this embodiment, when the display mode of the display image 25d is to be changed, the time axis scale of the capnogram waveform in the display image 25d can be changed. Accordingly, for example, displaying all of the capnogram waveforms for a single measurement unit side-by-side or displaying the enlarged mountain-shaped waveform constituting the capnogram waveform can be selectively conducted. Therefore, it is convenient for the user of the main body part 20 that analyzes the capnogram waveform, and is very suitable for easy and appropriate analysis of the capnogram waveform.
(g) In this embodiment, when the time axis scale of the capnogram waveform is to be changed, any one of a plurality of previously prepared time axis scales is selectively applied in response to the operation of the up button 27b. Therefore, an increase in processing load caused by changing the time axis scale of the capnogram waveform can be suppressed. In addition, changing the time axis scale only needs operation of the up button 27b, resulting in good operability.
(h) In this embodiment, when the display mode of the display image 25d is to be changed, the display reference position for arranging the capnogram waveforms side-by-side in the display image 25d can be changed. Therefore, for example, it is possible to easily and flexibly cope with selectively focusing on any one of the mountain-shaped waveforms constituting the capnogram waveform. As the result, it is very suitable for facilitating an easier and more appropriate analysis of the capnogram waveform.
(i) In this embodiment, when the display reference position for arranging the capnogram waveforms side-by-side is to be changed, any one of the waveform rising positions of the capnogram waveform respectively corresponding to each of N breathings constituting the single measurement unit is selectively applied as the display reference position in response to the operation of the down button 27c. Therefore, since the display reference position, which is a unified reference for the N capnogram waveforms (mountain-shaped waveforms), is applied, the order of the mountain-shaped waveforms of the respective capnogram waveform can be easily and appropriately rearranged. In addition, changing the display reference position only needs operation of the down button 27c, resulting in good operability.
(j) In this embodiment, the display part 25 can be controlled to selectively display any one of the capnogram waveforms respectively corresponding to each of N breathings constituting the single measurement unit in response to the operation in the operation part 27. Therefore, it is feasible to definitely focus on any one of the N capnogram waveforms and determine the state of the alveolar phase (plateau) in the focused waveform, which is very preferred for easy and appropriate analysis of the capnogram waveform.
(k) In this embodiment, the up button 27b and the down button 27c in the operation part 27 are also used as operation buttons for changing the display mode when the display part 25 conducts screen display. Therefore, even when the changeable display mode can be realized, the constitution of the operation part 27 can be prevented from being complicated. Further, the buttons 27b, 27c share roles, for example, the up button 27b has a role in changing the time axis scale of the capnogram while the down button 27c has a role in changing the display reference position for arranging the capnogram waveforms side-by-side, so that the operability for the user of the main body part 20 can be improved.
(l) In this embodiment, the timing of the exhalation start and the timing of the inhalation end are recognized based on the measurement result by the gas sensor 13, so that the icon image 25c which can distinguish the exhaled breath measurement state from the inhaled breath measurement state in the sensor probe 10 is displayed on the display screen of the display part 25 during the measurement of the carbon dioxide concentration. With such an icon image 25c being displayed, the exhaled breath measurement state can be distinguished from the inhaled breath measurement state during the breath test and the progress of the breath test can be easily and accurately informed, which makes it very convenient for the testee, the examiner and the like involved in the breath test.
(m) In this embodiment, at the time when the processing for a single measurement unit is completed, the sound output part 28 conducts sound output informing of the completion of the measurement, so that the testee, the examiner and the like involved in the breath test can be informed of the end of the test. Therefore, it is very convenient for the testee, the examiner and the like involved in the breath test since he/she can be easily and appropriately informed of the end of the test even without visually observing the screen display on the display part 25.

### <4. Modifications, etc.>

Although embodiments of the present invention have been specifically described, the present invention is not construed to be limited to the above-described embodiments, and various modifications may be made without departing from the gist of the invention.

The constitution in this embodiment delivers signals or power between the sensor probe 10 and the main body part 20 via the cable 15. Instead, another acceptable constitution may deliver signals or power via wireless communication or wireless power supply.

In addition, the breath test device according to the present invention can be widely applied to measurement of EtCO₂ of a subject including a person who visits medical institutions for diagnosis or treatment of COPD, a person who undergoes a regular medical examination, or a patient who is receiving medical treatment at home.

### Description of signs and numerals

- 1: Breath test device
- 10: Sensor probe
- 11: Case
- 12: Airway member
- 13: Gas sensor
- 14: Mouthpiece
- 20: Main body part
- 21: Measuring part
- 22: Count part
- 23: Detection part
- 24: Storage part
- 25: Display part
- 25c: Icon image
- 25d: Display image
- 26: Display control part
- 27: Operation part

## Claims

1. A breath test device provided with a sensor probe having a gas sensor for measuring an infrared absorptivity of a testee's breathing gas, and a main body part to which the sensor probe is communicably connected,
wherein the main body part comprises:
a measuring part which measures the concentration of carbon dioxide contained in the testee's exhaled breath based on the measurement result by the gas sensor for a period from the start to the end of a preset plurality of breathings which is taken as a measurement unit;
a storage part which stores the measurement result by the measuring part at least for the measurement unit;
a display part which displays the measurement result by the measuring part on a screen; and
an screen control part forming, as an image that the display part is controlled to display, a display image including all of capnogram waveforms arranged side-by-side on the screen based on the contents stored in the storage part, each capnogram waveform representing time-dependent change in the carbon dioxide concentration for the measurement unit.

2. The breath test device according to claim 1,
wherein the screen control part forms a display image and controls the display part to display the display image, at the time when the measuring part completes the measurement for the measurement unit.

3. The breath test device according to claim 1 or 2,
wherein the screen control part has a function to adjust a time axis scale of the capnogram waveform in the display image depending on the time required for the plurality of breathings constituting the measurement unit.

4. The breath test device according to any one of claims 1 to 3,
wherein the screen control part has a function to adjust the concentration range of the capnogram waveform in the display image depending on the measurement result of the carbon dioxide concentration by the measuring part.

5. The breath test device according to any one of claims 1 to 4,
wherein the main body part has an operation part which is operated by a user of the main body part, and the screen control part changes a display mode of the display image that the display part is controlled to display in response to an operation of the operation part.

6. The breath test device according to claim 5,
wherein the screen control part changes a time axis scale of the capnogram waveform in the display image when the display mode of the display image is to be changed.

7. The breath test device according to claim 6,
wherein the screen control part selectively applies any one of a plurality of previously prepared time axis scales in response to the operation of the operation part.

8. The breath test device according to any one of claims 5 to 7,
wherein the screen control part changes the time axis scale of the capnogram waveform for arranging the capnogram waveforms side-by-side in the display image when the display mode of the display image is to be changed.

9. The breath test device according to claim 8,
wherein the screen control part selectively applies, as the display reference position, any one of the waveform rising positions of the capnogram waveform respectively corresponding to each of N breathings constituting the measurement unit, in response to the operation of the operation part.

10. The breath test device according to claim 5,
wherein the screen control part controls the display part to selectively display any one of the capnogram waveforms respectively corresponding to each of N breathings constituting the measurement unit, in response to the operation of the operation part.

11. The breath test device according to any one of claims 1 to 10,
wherein the screen control part forms an icon image which can distinguish the exhaled breath measurement state from the inhaled breath measurement state in the sensor probe as an image that the display part is controlled to display, based on the measurement result by the gas sensor.

12. The breath test device according to any one of claims 1 to 11,
wherein, the main body part has a sound output part which conducts sound output informing of the completion of the measurement at the time when the measuring part completes the measurement for the measurement unit.
